# EUROPEAN PATENT SPECIFICATION

(11) **EP 3 143 952 B1**
(45) Date of publication and mention of the grant of the patent: **03.10.2018**
(21) Application number: 15822245.5
(22) Date of filing: 21.01.2015
(51) Int. Cl.: A61B 17/221, A61B 17/22

(54) **IRREGULARLY-SHAPED BALLOON-TYPE URETERAL STONE BLOCKAGE EXTRACTOR**
UNREGELMÄSSIG GEFORMTER BALLONARTIGER EXTRAKTOR FÜR HARNLEITERSTEINBLOCKADE
APPAREIL DE TYPE BALLONNET À FORME IRRÉGULIAIRE POUR EXTRAIRE DES CALCULS URÉTÉRAUX FAISANT OBSTRUCTION

(30) Priority: 15.07.2014 CN 201410334451
(43) Date of publication of application: 22.03.2017
(73) Proprietor: Second Military Medical University of the People's Liberation Army, Shanghai 200433 (CN)
(72) Inventor: SUN, Yinghao, Shanghai 200433 (CN)
(74) Representative: Valea AB
(86) International application number: PCT/CN2015/071190
(87) International publication number: WO 2016/008294

(56) References cited:
- WO-A1-97/38631
- WO-A2-2006/071855
- WO-A2-2009/086512
- CN-A- 1 972 636
- CN-A- 102 370 506
- CN-A- 104 095 668
- CN-U- 203 970 487
- JP-A- 2008 194 167
- US-A- 5 112 347
- US-A- 5 827 324
- US-A- 5 871 454
- US-A- 6 001 118
- US-A1- 2005 228 417

## Description

### TECHNICAL FIELD

The present invention relates to medical devices. More specifically, the present invention relates to an irregularly-shaped balloon-type ureteral stone blockage extractor.

### BACKGROUND

The incidence rate of urinary stone is up to 5%-10%, which may occur at any part of kidney, bladder, ureter and urethra. Among which, kidney and ureteral stone is the most common. Ureteroscopic lithotripsy for the treatment of urinary stone fragments within the natural human body lumen. It is a kind of minimally invasive treatment and can accurately fragment stone, so it is currently one of the main treatment means. However, current ureteroscopic lithotripsy has some deficiencies: 1. stone and stone fragments in the intermediate segment and upper segment of ureter can be easily flushed back to kidney by the recoil force of the injected fluid or the fragmenting tool; 2. The residual stone fragments can't be extracted out efficiently.

An important means for preventing ureteral stone from being flushed back to the kidney is to occlude the upper side of ureteral stone by tools. There are some clinic ureter occluding device which also can be used to extract stone. However, they still have shortcomings.

Basket is commonly used for occluding and extracting stone. The basket will expand as a mesh after moving across to the upper side of the stone, which will prevent the stone fragments from drifting upward and grasp the small stone fragments. But the basket is of large volume and is not convenient to be introduced into ureter. It may push the stone fragments to drift. Meanwhile, the basket need to be repeatedly introduced into the ureter as only a small amount of stone can be extracted at a once, and there is risk for residue stone to shift when the water was injected again and again. In addition, sharp stone can be easily pushed out through the holes of basket and scratch the ureter wall during dragging procedure, and may bring complication.

Another occluding method is to use film or balloon to occlude the ureter lumen as much as possible. For example, Chinese Patent CN 200910057068.2, published on Oct 14, 2009, discloses an occluding device for occluding obstacles in body lumen. The occluding device comprises a guide wire, a catheter and an occluding object. One distal end of the guide wire passes through the catheter and the body lumen. The distal end is fixed to the guide wire. Pull the proximal end of the guide wire, the expanded occluding object is axially compressed to an emboliform occluding object. The occluding device is flat film. The occluding device prevents stone from shifting upwards by forming emboliform occluding object, and the occluding device can be used for dragging a plurality of stone fragments towards the bladder after the surgery. However, the occluding device can't totally occlude the ureter lumen, especially during the stone dragging process, because the occluding device is prone to deform due to water injection into the working channel of the endoscope. Thus, stone will slip via the space between the occluding device and the ureter wall, or stone and the occluding device may be trapped in the ureter and the ureter mucosa will be injured if there are large quantity of stone. Furthermore, the existing film-like occluding device or balloon often has large volume, so it is not easy to be introduced into ureter like basket, and is more easily be pushed back to kidney.

WO 2009/086512 describes a prior art exhangeable guide-wire with balloon for foreign body extractor, wherein a fitting groove is provided at a distal end of the guide-wire and an annular balloon is provided on the fitting groove.

US 5,827,324 describes an emboli capturing system including a guide wire, an expandable member and an emboli captiring device; wherein the emboli capturing deviuce is coupled to a distal portion of the guidewire and configured to deploy radially ooutwardly relative to the guidewire upon expansion of the expandable member.

As mentioned above, there is an urgent need for a ureteral stone blockage extractor which can be conveniently introduced into ureter, not easy to cause dislodges of stone, not easy to deform when crushed stone is being removed, can fully occlude the ureteral stone, not easy for trapping stone to injure ureter wall, and can extract stone more conveniently. But there is no such kind of devices been reported at present.

### SUMMARY

To overcome deficiencies in the prior art, there is provided an irregular-shaped balloon-type ureteral stone blockage extractor.

To achieve the above purpose, the technical solution provided by the present invention is:
An irregular-shaped balloon-type ureteral stone blockage extractor, according to claim 1, comprising a hollow operating rod with an open proximal end and a closed distal end, a fitting groove is provided at a distal end segment of the operating rod, an annular balloon is provided on the fitting groove, an occluding film is provided on the periphery of the balloon, the internal part of the balloon is intercommunicated with a cavity of the operating rod, wherein the occluding film is fixed along the outer periphery of the largest diameter of the balloon.

The fitting groove surrounds the operating rod by one circle, the balloon surrounds the fitting groove by one circle, and the plane corresponding to the diameter of the balloon is perpendicular to the longitudinal axis of the operating rod.

The occluding film surrounds the periphery of the balloon by at least one circle.

The occluding film includes a single film, or the occluding film consists of a plurality of valves.

The occluding film includes a plurality of valves, the valves are overlapped configured and surrounds the balloon by a plurality of circles.

The lower edge of the occluding film is inclined to the proximal end of the operating rod.

There is a through-hole on the connecting part between the operating rod and the balloon.

The through-hole is configured to surround the operating rod.

The top end of the distal end segment of the operating rod is provided with a guiding tip which has a curved surface.

The advantages of the present invention:
1. An occluding film is provided on the periphery of the balloon which is not easy to trap stone, so the diameter of the whole extractor comprised of balloon and occluding film can be designed to be slightly larger than the diameter of the ureter. Thus, the ureter lumen can be fully occluded to prevent stone from slipping and missing, and stone can be extracted by one time. Furthermore, the incidence of stone struck in the ureter can be reduced.
2. Both the balloon and occluding film are made of plastic film, so they can be compressed to a small volume. There is also fitting groove provided on the operating rod, so both the balloon and occluding film can be compressed in the fitting groove to ease the introduction of the extractor. Also the stone can not be pushed back to the kidney, and eliminate trauma to the ureter wall.
3. The lower edge of the occluding film is inclined to the proximal end of the operating rod, so that the occluding film can form a space together with the balloon. The space can contain parts of stone fragments, minimize the friction against the ureter. The design that the occluding film is inclined to the proximal end of the operating rod and the occluding film is fixed along the outer periphery of the largest diameter of the balloon will ensure that the occluding film can keep extending and is not easy to deform when pushed by the poured liquid in the working channel of endoscope.
4. The top end of the distal end segment of the operating rod is provided with a guiding tip which has a curved surface, so that the extractor will not injure the ureter wall when the extractor is introduced into the ureter.

### BRIEF DESCRIPTION OF THE DRAWINGS

Figure 1 is a structure diagram of one embodiment of irregular-shaped balloon-type ureteral stone blockage extractor.
Figure 2 is an enlarged cutaway view of A in Figure 1.
Figure 3 is B-B cross-sectional view of Figure 2.

### DETAILED DESCRIPTION

Detailed description according to the figures will be given combined with embodiments.

Labels and components related to the figures are as follows:

| | |
|---|---|
| 1. Operating rod | 11. Proximal end segments of the operating rod |
| 12. Distal end segment of the operating rod | 2. Handle |
| 3. Guiding tip | 4. Fitting groove |
| 5. Balloon | 6. Occluding film |
| 7. Through-hole | |

### Embodiment 1

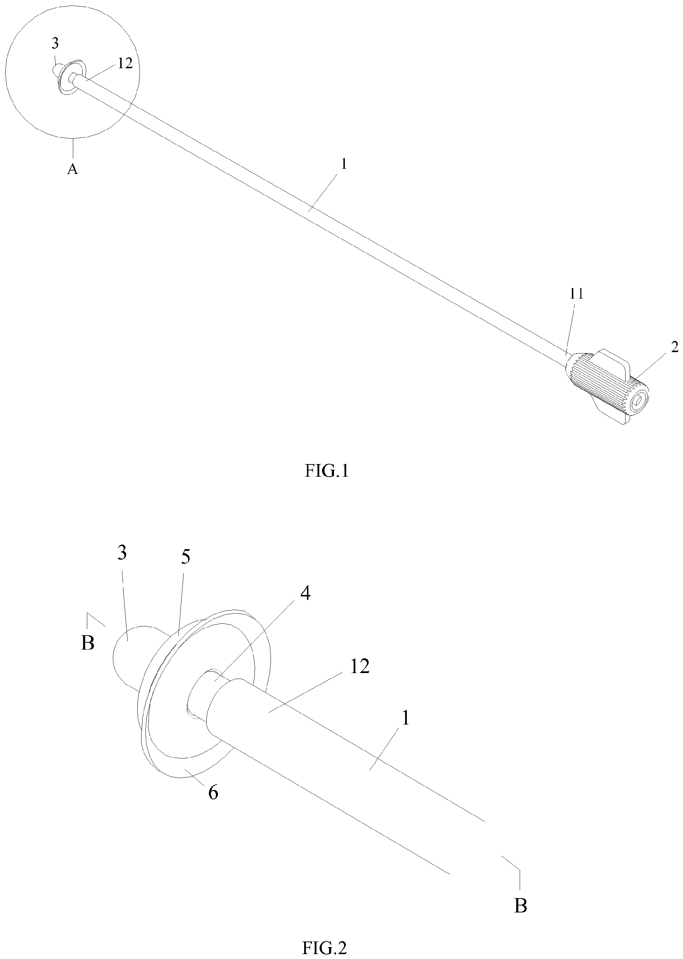

Referring to Figure 1, Figure 1 is a structure diagram of one embodiment of irregular-shaped balloon-type ureteral stone blockage extractor. The ureteral stone blockage extractor is provided with an operating rod 1. The operating rod 1 is cylinder-shaped hollow tube with open proximal end and closed distal end. A handle 2 is provided on the periphery of the proximal end segments of the operating rod 11. The top end of the distal end segment of the operating rod 12 is provided with hemispherical guiding tip 3.

Referring to Figure 2, Figure 2 is an enlarged cutaway view of A in Figure 1. A fitting groove 4 is provided at the back side of the guiding tip 3 of the distal end segment of the operating rod 12. The fitting groove 4 surrounds the operating rod 1 for one circle. The fitting groove 4 is provided with an annular balloon 5. The balloon 5 surrounds the fitting groove 4 for one circle, and the plane corresponding to the diameter of the balloon is perpendicular to the operating rod 1. An occluding film 6 is provided on the periphery of the balloon 5. The occluding film 6 includes a single film, and surrounds the balloon 5 for one circle.

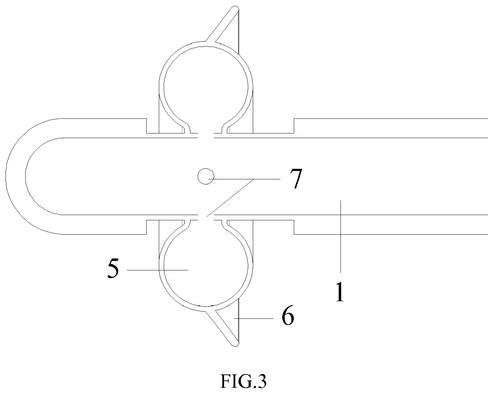

Referring to Figure 3, Figure 3 is B-B cross-sectional view of Figure 2. The occluding film 6 is fixed along the outer periphery of the largest diameter of the balloon 5, and the lower edge of the occluding film 6 is inclined to the proximal end of the operating rod 1. There is provided a through-hole 7 on the connecting part between the operating rod 1 and balloon 5. A plurality of through-holes 7 are configured to surround the operating rod 1.

To be specified:
In one embodiment of the invention, the balloon 5 of the extractor is relieved before it is used, that is factory state. Both the balloon 5 and the occluding film 6 fold and are affixed to the fitting groove 4, so that the diameter of fitting groove 4 is not larger than the diameter of the operating rod 1.

As used in this disclosure, the term "proximal end" means the nearer end to the operator during the surgical procedure, the term "distal end" means the farer end to the operator during the surgical procedure. The term "proximal end segments of the operating rod 11" means the segment that is near the proximal end, and the term "distal end segment of the operating rod 12" means the segment that is near the distal end. Both the proximal end segments of the operating rod 11 and the distal end segment of the operating rod 12 don't have specific length. In one embodiment of the present invention, the distal end segment of the operating rod 12 have a length that allow at least the guiding tip 3 and the fitting groove 4.

The balloon 5 is used for occluding stone. The balloon 5 is sealed and solid occluding object, and is not easy to deform when flushed by liquid. The occluding film 6 is further used for occluding the gap that is possibly formed between the balloon 5 and the inner wall of the ureter. Since the occluding film 6 will not cause great friction force to the ureter wall, so the diameter of the whole extractor constructed by balloon 5 and occluding film 6 can be designed to be slightly larger than the diameter of the ureter to enable complete occlusion, prevention for slipping and extraction of stone at one time. In one preferred embodiment, the diameter of the whole extractor constructed by balloon 5 and occluding film 6 is between about 4mm and about5mm. Furthermore, the contact surface between the occluding film 6 and the inner wall of the ureter is very small, which will greatly reduce the incidence of stone fragments' concentration in ureter. The occluding film 6 is not limited to be designed to surround the balloon 5 by one circle. The occluding film 6 can be designed to surround the balloon 5 by a plurality of circles. The occluding film 6 can be a complete single film, or can comprised of a plurality of valves. For example, a plurality of fan-shaped valves are interlocked or overlapped to surround the balloon 5 by a plurality of circles. All the above structures of the occluding film 6 can fully occlude the ureter, and reduce the incidence of stone concentration, provide space for containing stone fragments, thus eliminate trauma to the inner wall of the ureter. In one preferred embodiment, the occluding film 6 is fixed along the outer periphery of the largest diameter of the balloon 5, and the lower edge of the occluding film 6 is inclined to the proximal end of the operating rod 1, which can ensure complete occlusion and is not easy for stone fragments slipping and concentration, and the occluding film 6 can keep extending and is not easy to deform when pushed by the poured liquid in the working channel of endoscope. The guiding tip 3, balloon 5 and occluding film 6 can be independent elements, and they can be fixed on the operating rod 1 by welding, sticking, or other methods. They can also be one-batch formed together with the operating rod 1. The material of balloon 5 and occluding film 6 can be selected from TPU, Pebax, FEP, ETFE, TPFE etc. These materials have small volume, and has good malleability and formability. The fitting groove 4 is used for containing relieved balloon 5 and occluding film 6, so that the overall diameter of the extractor is comparatively small, and the extractor can easily steer clear of the stone when being introduced. The extractor is also not easy to cause dislodge of stone or injure the inner wall of the ureter. The fitting groove 4 is not limited to be designed to surround the operating rod 1 by one circle. It can be designed as multiple stripped fitting groove surrounding the operating rod 1 and are configured with space intervals. The hollow lumen of the operating rod 1 is used for injecting gas or liquid from the proximal end, and the gas or liquid will go through the through-hole 7 to inflate the balloon 5. The through-hole 7 is used for connecting the internal part of the balloon 5 and the lumen of the operating rod 1. The through-hole 7 can be circular hole, square hole, or other shapes of hole, and the through-hole 7 can be configured in any forms. In one preferred embodiment, the guiding tip 3 is designed as hemispherical shape. It can also be any other curved surface shape that can introduce the extractor into the ureter without any injuries to the ureter wall. The proximal end of the operating rod 1 is used to connect injector. The proximal end can comprise normally closed type single opening exhaust valve to seal the lumen of the operating rod 1 and keep the balloon 5 in inflation state.

An exemplary use method of the irregularly-shaped balloon-type ureteral stone blockage extractor includes: when the ureteroscopic lithotripsy is being executed, the working channel of the endoscope shall be formed. Input the endoscope into the working channel, unfold the factory state package of the extractor, hold the handle 2 and input the guiding tip 3 of the operating rod 1 into the ureter, steer clear of the stone to get to the upper side of the stone. Then infuse gas or liquid into the lumen of the operating rod 1 through the proximal end of the operating rod 1. The infused gas or liquid gets through the through-hole to enter the lumen of the balloon 5, which expand the balloon 5 to annular shape. The occluding film 6 which will subsequently be extended and tightly attach to the inner wall of the ureter. Then infuse liquid through the working channel of the endoscope to the ureter while dragging the operating rod 1 to the proximal end. Stone fragments will be flushed by continuous infused liquid into the space between the balloon 5 and the occluding film 6, or flow to the bladder while the liquid flushed and stirred and the operating rod 1 is slowly being dragged. The ureteral stone blockage extractor has a small dimension, can be easily introduced into a ureter, would not push a stone in reverse towards a kidney and eliminate trauma to the ureter wall, can fully occlude the ureter, effectively prevents any stone and stone fragments from being flushed back into the kidney by injected liquid, is not prone to get stone stuck in ureter, does not deform easily or rub against the ureter wall during a stone extraction process, and can remove all stone in a single run, thus achieving the goal of efficient stone removal.

The irregularly-shaped balloon-type ureteral stone blockage extractor disclosed may not only be used for occlusion and removal of ureteral stone, but also for occlusion and removal within other kind of body lumens and other obstructions, including, but not limit to occlusion and removal of kidney stone, occlusion and removal of embolus of vessel, occlusion and stop of trocar puncture hole hemorrhage of vessel, occlusion and removal of obstructions in trachea or intestinal tract.

The above disclosed is only a preferred embodiment of the present invention, ordinary skills in the art can make multiple improvements and changes within the scope of the appended claims.

## Claims

1. An irregular-shaped balloon-type ureteral stone blockage extractor comprising a hollow operating rod (1) with an open proximal end (11) and a closed distal end (12), wherein a fitting groove (4) is provided at a distal end segment of the operating rod (1), an annular balloon (5) is provided on the fitting groove (4), an occluding film (6) is provided on the periphery of the balloon (5), the internal part of the balloon (5) is intercommunicated with a cavity of the operating rod (1), wherein, the occluding film (6) is fixed along the outer periphery of the largest diameter of the balloon (5), the balloon (5) is used for occluding stone, and the occluding film (6) is further used for occluding the gap that is formed between the balloon (5) and the inner wall of a ureter.

2. The irregular-shaped balloon-type ureteral stone blockage extractor according to claim 1, wherein the fitting groove (4) surrounds the operating rod (1) by one circle, the balloon (5) surrounds the fitting groove (4) by one circle, and the plane corresponding to the diameter of the balloon (5) is perpendicular to the longitudinal axis of the operating rod (1).

3. The irregular-shaped balloon-type ureteral stone blockage extractor according to claim 1, the occluding film (6) surrounds the periphery of the balloon (5) by at least one circle.

4. The irregular-shaped balloon-type ureteral stone blockage extractor according to claim 3, the occluding film (6) includes a single film, or the occluding film (6) consists of a plurality of valves.

5. The irregular-shaped balloon-type ureteral stone blockage extractor according to claim 4, the occluding film (6) includes a plurality of valves, the valves are overlapped configured and surrounds the balloon (5) by a plurality of circles.

6. The irregular-shaped balloon-type ureteral stone blockage extractor according to claim 1, the lower edge of the occluding film (6) is inclined to the proximal end of the operating rod (11).

7. The irregular-shaped balloon-type ureteral stone blockage extractor according to claim 1, there is a through-hole (7) on the connecting part between the operating rod (1) and the balloon (5).

8. The irregular-shaped balloon-type ureteral stone blockage extractor according to claim 7, the through-hole (7) is configured to surround the operating rod (1).

9. The irregular-shaped balloon-type ureteral stone blockage extractor according to claim 1, the top end of the distal end segment of the operating rod (12) is provided with a guiding tip (3) which has a curved surface.

## Patentansprüche

1. Unregelmäßig geformter Harnleitersteinblockierungsextraktor vom Ballontyp, umfassend einen hohlen Operationsstab (1) mit einem offenen proximalen Ende (11) und einem geschlossenen distalen Ende (12), wobei an einem distalen Endsegment des Operationsstabes (1) eine Passnut (4) vorgesehen ist, an der Passnut (4) ein ringförmiger Ballon (5) vorgesehen ist, am Umfang des Ballons (5) eine Verschlussfolie (6) vorgesehen ist, der innere Teil des Ballons (5) mit einem Hohlraum des Operationsstabes (1) in Verbindung steht, wobei die Verschlussfolie (6) entlang des äußeren Umfangs des größten Durchmessers des Ballons (5) befestigt ist, der Ballon (5) zum Verschließen von Stein verwendet wird und die Verschlussfolie (6) weiter zum Verschließen des zwischen dem Ballon (5) und der Innenwand eines Harnleiters gebildeten Spaltes verwendet wird.

2. Unregelmäßig geformter Harnleitersteinblockierungsextraktor vom Ballontyp nach Anspruch 1, wobei die Passnut (4) den Operationsstab (1) um einen Kreis umgibt, der Ballon (5) die Passnut (4) um einen Kreis umgibt und die dem Durchmesser des Ballons (5) entsprechende Ebene senkrecht zur Längsachse des Operationsstabes (1) ist.

3. Unregelmäßig geformter Harnleitersteinblockierungsextraktor vom Ballontyp nach Anspruch 1, wobei die Verschlussfolie (6) den Umfang des Ballons (5) um mindestens einen Kreis umgibt.

4. Unregelmäßig geformter Harnleitersteinblockierungsextraktor vom Ballontyp nach Anspruch 3, wobei die Verschlussfolie (6) eine einzige Folie enthält oder die Verschlussfolie (6) aus einer Vielzahl von Ventilen besteht.

5. Unregelmäßig geformter Harnleitersteinblockierungsextraktor vom Ballontyp nach Anspruch 4, wobei die Verschlussfolie (6) eine Vielzahl von Ventilen enthält, die Ventile überlappend ausgebildet sind, und den Ballon (5) mit einer Vielzahl von Kreisen umgibt.

6. Unregelmäßig geformter Harnleitersteinblockierungsextraktor vom Ballontyp nach Anspruch 1, wobei die Unterkante der Verschlussfolie (6) zum proximalen Ende des Operationsstabs (11) geneigt ist.

7. Unregelmäßig geformter Harnleitersteinblockierungsextraktor vom Ballontyp nach Anspruch 1, wobei es am Verbindungsteil zwischen dem Operationsstab (1) und dem Ballon (5) eine Durchgangsbohrung (7) gibt.

8. Unregelmäßig geformter Harnleitersteinblockierungsextraktor vom Ballontyp nach Anspruch 7, wobei die Durchgangsbohrung (7) so ausgebildet ist, dass sie den Operationsstab (1) umgibt.

9. Unregelmäßig geformter Harnleitersteinblockierungsextraktor vom Ballontyp nach Anspruch 1, wobei das obere Ende des distalen Endsegmentes des Operationsstabs (12) mit einer Führungsspitze (3) versehen ist, die eine gekrümmte Oberfläche aufweist.

## Revendications

1. Appareil de type ballonnet à forme irrégulière pour extraire des calculs urétraux faisant obstruction comprenant une tige d'actionnement creuse (1) avec une extrémité proximale ouverte (11) et une extrémité distale fermée (12), dans lequel une rainure de montage (4) est prévue au niveau d'un segment d'extrémité distale de la tige d'actionnement (1), un ballonnet annulaire (5) est prévu sur la rainure de montage (4), un film d'occlusion (6) est prévu sur la périphérie du ballonnet (5), la partie interne du ballonnet (5) est en communication avec une cavité de la tige d'actionnement (1), dans lequel, le film d'occlusion (6) est fixé le long de la périphérique extérieure du diamètre le plus grand du ballonnet (5), le ballonnet (5) est utilisé pour l'occlusion du calcul, et le film d'occlusion (6) est utilisé en outre pour l'occlusion de l'espace qui est formé entre le ballonnet (5) et la paroi intérieure d'un urètre.

2. Appareil de type ballonnet à forme irrégulière pour extraire des calculs urétraux faisant obstruction selon la revendication 1, dans lequel la rainure de montage (4) entoure la tige d'actionnement (1) par un cercle, le ballonnet (5) entoure la rainure de montage (4) par un cercle, et le plan correspondant au diamètre du ballonnet (5) est perpendiculaire à l'axe longitudinal de la tige d'actionnement (1).

3. Appareil de type ballonnet à forme irrégulière pour extraire des calculs urétraux faisant obstruction selon la revendication 1, le film d'occlusion (6) entoure la périphérie du ballonnet (5) par au moins un cercle.

4. Appareil de type ballonnet à forme irrégulière pour extraire des calculs urétraux faisant obstruction selon la revendication 3, le film d'occlusion (6) comprend un seul film, ou le film d'occlusion (6) consiste en une pluralité de valves.

5. Appareil de type ballonnet à forme irrégulière pour extraire des calculs urétraux faisant obstruction selon la revendication 4, le film d'occlusion (6) comprend une pluralité de valves, les valves sont configurées de manière à se chevaucher et entoure le ballonnet (5) par une pluralité de cercles.

6. Appareil de type ballonnet à forme irrégulière pour extraire des calculs urétraux faisant obstruction selon la revendication 1, le bord inférieur du film d'occlusion (6) est incliné par rapport à l'extrémité proximale de la tige d'actionnement (11).

7. Appareil de type ballonnet à forme irrégulière pour extraire des calculs urétraux faisant obstruction selon la revendication 1, il y a un trou traversant (7) sur la partie de liaison entre la tige d'actionnement (1) et le ballonnet (5).

8. Appareil de type ballonnet à forme irrégulière pour extraire des calculs urétraux faisant obstruction selon la revendication 7, le trou traversant (7) est configuré pour entourer la tige d'actionnement (1).

9. Appareil de type ballonnet à forme irrégulière pour extraire des calculs urétraux faisant obstruction selon la revendication 1, l'extrémité supérieure du segment d'extrémité distale de la tige d'actionnement (12) est dotée d'une pointe de guidage (3) qui a une surface incurvée.
